# EUROPEAN PATENT SPECIFICATION

(11) **EP 3 801 306 B1**
(45) Date of publication and mention of the grant of the patent: **10.01.2024**
(21) Application number: 19815782.8
(22) Date of filing: 05.06.2019
(51) Int. Cl.: A61B 17/3207, A61B 17/221, A61B 17/00, A61B 17/22, A61M 25/10

(54) **SCORING DEVICES AND CATHETER SYSTEMS FOR CONTROLLED VESSEL LESION DISSECTION**
SCHNEIDVORRICHTUNGEN UND KATHETERSYSTEME FÜR KONTROLLIERTE GEFÄSSLÄSIONSDISSEKTION
DISPOSITIFS DE COUPE ET SYSTÈMES DE CATHETER POUR DISSECTION DE LÉSION VASCULAIRE CONTRÔLÉE

(30) Priority: 08.06.2018 US 201862682642 P
(43) Date of publication of application: 14.04.2021
(73) Proprietor: SurModics, Inc., Eden Prairie, MN 55344-3523 (US)
(72) Inventor: SHAKED, Yoav, 4069500 Mishmeret (IL); MEERKIN, David, 9223309 Jerusalem (IL); BENARY, Raphael, 6949412 Tel Aviv (IL)
(74) Representative: Murgitroyd & Company
(86) International application number: PCT/IB2019/054685
(87) International publication number: WO 2019/234659

(56) References cited:
- EP-A1- 2 455 017
- WO-A1-2018/094077
- US-A- 5 176 693
- US-A1- 2004 243 158
- US-A1- 2013 041 391
- US-A1- 2013 041 391
- US-A1- 2013 190 789
- US-A1- 2015 100 079
- US-A1- 2016 262 789
- US-A1- 2017 319 231

## Description

### FIELD OF THE DISCLOSURE

Embodiments of the present disclosure relate to systems, devices and methods for treating a vessel or heart valve using focused force, to aid in controlled dissection of lesions in or adjacent the structure thereof.

### BACKGROUND

Balloon dilation (angioplasty) is a common medical procedure used to increase the luminal dimensions of stenotic vessels, most commonly arteries narrowed by atherosclerotic plaque. The angioplasty procedure works by threading a catheter, tipped with a deflated dilatation balloon, through the vascular system to the target stenotic site. Once at the appropriate site, the balloon is inflated which causes radial pressure to be applied to the inner wall of the vessel. This helps redistribute plaque on the vessel wall to a more favorable (less occlusive) configuration, and widens the stenosed region to enable better blood flow. Unfortunately, in many cases after balloon dilatation, the plaque and the blood vessel walls eventually redistribute to an unfavorable configuration again, often allowing vessel re- occlusion.

In the past, to overcome some of these issues, angioplasty balloons included blades or scoring elements.

However, these disclosures provide limited scoring functionality, as the scoring/blade elements do not address extended lesions, large vessel diameter, heart valves, and bifurcation.

US 2013/041391 A1 concerns a medical device assembly for incising a stenosis in a blood vessel which includes an incising device, a tubular sheath, and a catheter having an inflatable balloon mounted thereon. The incising device includes a self-expanding scaffold having a plurality of cutting elements projecting radially outward therefrom, and an elongate member extending proximally from the self-expanding scaffold to be manipulated by a user. The self-expanding scaffold is positionable in the lumen of the sheath in a contracted configuration and deployed out of the lumen of the sheath to permit the self-expanding scaffold to expand to an expanded configuration. The inflatable balloon of the catheter is configured to be advanced distally into an interior of the self-expanding scaffold through a proximal opening of the self-expanding scaffold, and inflation of the inflatable balloon urges the cutting elements radially outward to incise a stenosis.

Similarly, US 2017/319231 A1 also describes a medical device that includes an expandable-and-contractible member capable of radially expanding and radially contracting, an incising member disposed on an outer circumference of the expandable-and-contractible member and having a hollow portion, a core wire inserted in the hollow portion, a distal tip, and a ring.

US 2016/262789 A1 also relates to the scoring of a stenotic region within a vessel of a patient. An assembly comprising a scoring member and a deployment member is disclosed. The scoring member can include an elongate push structure and a tubular, self-expanding scaffold. The elongate push structure can be positioned proximal of the self-expanding scaffold and, at its distal end portion, eccentrically coupled with a proximal end portion of the self-expanding scaffold. The deployment member can be removably positioned within a central lumen of the self-expanding scaffold and detachably engageable with a portion of the self-expanding scaffold. The self-expanding scaffold can be movable between a downsized configuration, when receiving axial tension from the deployment member, and an expanded configuration, in the absence of axial tension. The assembly can further comprise means for detachably engaging the deployment member and the self-expanding scaffold and means for detachably engaging the deployment member and the elongate push structure.

EP 2455017 A1 concerns treatment of a lesion in a body lumen to enlarge a passageway in the lumen. Disclosed here is an assembly of a cutting member and a tracking member configured for insertion through a first lumen of a catheter. A coupler may be used to connect the cutting member to the tracking member.

### SUMMARY OF SOME OF THE EMBODIMENTS

The invention is the scoring device and the catheter system as defined in the appended claims. The methods disclosed do not form part of the claimed invention.

It is therefore an object of some of the embodiments of the present disclosure to provide an enhanced scoring catheter system and scoring device and , in some embodiments, improved maneuverability and multiple treatment options (for example).

Systems, devices and methods for treating a stenosed vessel or heart valve are disclosed. In some embodiments, a cutting/scoring assembly/device for treating a stenosed vessel/heart valve is provided and includes an elongated element having multiple longitude wires arranged at least at a distal end. In some embodiments, a main elongated element can be a single wire (or intertwined multiple wires) that continues to the distal tip of the device, with additional wires connecting to the tip at their distal ends, and connecting at the main wire at their (additional wires) proximal ends. In some embodiments, the distal tip (which can perform as a guide wire rail), is connected to the multiple wires, which can be positioned at a rotational distance of each other. That is, the wires, in some embodiments, are radially spaced from one another; moreover, in some embodiments, the radial distance need not be equal. It is noted that the term "scoring" throughout the description and claims refers to any kind of reduction in size or any modification in shape or form of an occlusion in a body lumen, such as but not limited to, cutting, scoring, fracturing, pulverizing, breaking, grinding, chopping and the like.

For example, in some embodiments, using for example two scoring elements (e.g., wires), the radial spacing can range from 0 degrees (i.e., the wires extend from the same point), up to, in some embodiments, 180 degrees (i.e., the wires extend from opposite ends, radially). For a three scoring element device/system, the radial spacing between any two wires range can vary from 0 to 120 degrees. Also, the wires need not be distributed equally in the radial direction.

In some embodiments, the multiple wires can emerge from the same site/area/portion on the distal tip of the device. The device can be used in advance of a balloon catheter (which may be a standard balloon catheter), and both can be advanced separately, or in some embodiments, simultaneously, over the same guide wire. In some embodiments, the balloon catheter is configured to push/advance the distal tip of the cutting assembly through a blood vessel. In such embodiments, the proximal portions of the multiple wires are configured to fall alongside the length of the balloon. Inflation of the balloon causes the multiple distal longitude wires positioned alongside the balloon catheter to be forced up against the vessel wall providing a "scoring element" for, according to some embodiments, localized controlled longitudinal vessel incision.

In some embodiments, the scoring elements may be configured as a braid. For example, in some embodiments, the distal and proximal ends of the braided section can be brought towards the one another, and the braided section can be configured to increase in diameter (e.g., similar to a Chinese finger trap). In such embodiments, a balloon catheter can be inserted into the expanded braided area, and upon the ends being allowed to move away from one another, the scoring elements resume the earlier (smaller) diameter (i.e., "locking" on the balloon).

According to some embodiments of the present disclosure, a device for introduction into a vessel is provided which includes a first (e.g., main) elongated wire at a length of between about 120 cm and 180 cm, and in some embodiments, up to 300 cm (various embodiments include various specific length ranges within such ranges, including, for example, 120 cm - 170 cm, 130 cm - 160 cm, etc.). In some embodiments, this first wire acts as a body of the device. In some embodiments, the first wire includes a diameter of between about 0.25mm (0.010 inches) to about 0.97mm (0.038 inches) (various embodiments include ranges of diameters within this range), and is connected at a distal end to one or more longitude wires. These one or more distal wires, in some embodiments, include a length of between about 10 mm and 300 mm, and in some embodiments, to about 400 mm. In some embodiments, the one or more distal wires correspond to multiple distal longitude wires, which may all be connected into a distal tapered tip. Such embodiments can be used in conjunction with a guide wire and a balloon catheter (the device with at least one of the guide wire and a balloon catheter correspond to a system embodiment(s) of the present disclosure. The expandable scoring device of any of the embodiments of the invention has the capability of being assembled with a wide range of lengths and diameters of balloon catheters or other similar stenosed vessel treatment devices. Accordingly, the scoring device is a universal, one-size-fits-all device for coupling with balloon catheters or other similar stenosed vessel treatment devices.

A non-claimed method for treating a vessel is also described. The method includes providing a device such as described above, using said device, for example, with multiple distal longitudinal wires, inserting a tracking guidewire into the vessel, back-loading the tracking guidewire into the distal tip of the device, advancing the device over the tracking guidewire until the distal end of the device is adjacent to the lesion, advancing a balloon catheter over the tracking guidewire until the distal end of the balloon catheter reaches a proximal end of the distal tip of the device, and then inflating the balloon so as to push at least the distal longitude wires against different sides of a lesion in the vessel.

In some embodiments, a stenosed vessel treatment device is provided and includes an elongated element comprising a plurality of wires arranged longitudinally along a length from a first proximal end and a second distal end, and a tip sized and configured so as to enable the device to traverse the interior of a blood vessel, the tip including a central opening configured to receive a guide wire. The plurality of wires are spaced apart from one another along at least a portion of the length between the proximal and distal ends of the device, and the plurality of wires are arranged in a generally cylindrical shape for receiving a balloon catheter therebetween, such that, the plurality of wires are arranged along the outside of the balloon, whereby upon inflation of the balloon, the plurality of wires are forced against a lesion adhered to the wall of a blood vessel.

The above noted embodiments may include one and or another of the following features, structure, function, steps, and/or clarifications which, together in various combinations, provide yet further embodiments of the disclosure: a) the plurality of wires comprise a cutting assembly; b) the device includes a length of between about 120 cm and about 180 cm; c) a diameter of at least one of the at least two wires is between about 0.25mm (0.010 inches) and about 0.97mm (0.038 inches); d) one or more of the plurality of wires are connected at their distal ends to the tip; e) a first of the plurality of wires comprises a main wire configured to perform as a body of the device; f) the first wire may be connected at the distal end to at least one or more of the remainder of the plurality of wires; and/or g) the one or more remainder of the plurality of wires include a length of between about 10 mm and about 300 mm, and in some embodiments, to about 400 mm; h) the plurality of wires are intertwined to form a helix; and/or i) the plurality of wires comprise two or three wires.

In some embodiments, a scoring device configured to fit in-line over a surgical balloon catheter system is provided which comprises a cylindrically-shaped main body having a plurality of cutting elements circumferentially spaced apart and configured to fit over a balloon of a surgical balloon catheter system and expand therewith in the radial direction and retract the same with the inflation and deflation thereof, respectively. The device also can optionally include a tip portion configured to radial expand a second amount so as to accept a tip of the balloon catheter system. The scoring device may be made of a shape memory and/or superelastic material, such as but not limited to, nitinol. The shape memory and/or superelastic material properties of the scoring device, when positioned on the balloon catheter, may provide forces on the balloon catheter that assist in the deflation of the balloon catheter. It is noted that the tip is an abutment structure through which at least a portion of the balloon cannot pass. Alternatively, the scoring device may be made from other medically safe materials, such as but not limited to, stainless steel alloys, aluminum alloys, titanium alloys and others.

The above noted embodiments may include one and or another of the following features, structure, function, steps, and/or clarifications which, together in various combinations, provide yet further embodiments of the disclosure: a) the tip portion comprises a flexible composite material, which may be a polymeric material, and/or a metal; b) a proximal collar, wherein: c) the proximal collar may be configured as a cylindrical band, where the cylindrical band includes an open portion, where the open portion is between: d) about 1 deg. and about 179.9 deg. of the circumference of the band, e) about 10 deg. and about 120 deg. of the circumference of the band, or f) about 30 deg. and about 90 deg. of the circumference of the band; g) the open portion may be configured so as to allow the proximal collar to snap on or otherwise fit to a shaft of the balloon catheter system; h) the proximal collar may be configured to snap or fit to the shaft such that it remains affixed to the shaft while also being slidably movable along the shaft in the axial direction; i) a wire control element, where the wire control element is connected to collar such that the control wire extends proximally, and/or a proximal end of the control wire is accessible to the operator either directly or via another device; j) the tip portion include a plurality of cells which are configured to expand radially outward upon the application of a force and retract when the force is removed; k) the collar being configured to fit a range of balloon catheter shaft and corresponding diameters; 1) the proximal collar comprises an expandable section which is configured to fit a range of catheter shafts and corresponding diameters thereof; m) the proximal collar includes an expandable section configured to radially expand upon the application of a radial force; n) the proximal collar being configured to fit over the shaft of the balloon catheter system so as to prevent dislodgement of the scoring device and the axial movement of the proximal collar along the catheter shaft allows the scoring device to shorten in length when the scoring elements are forced radially outward by the inflation of the balloon of the balloon catheter system; p) the tip of the balloon catheter system is positioned proximal to the tip portion; q) the cutting elements are extend axially along the catheter shaft for a distance between about 10 mm and about 300 mm, and in some embodiments, to about 400 mm; r) a radially intermediate expandable section located between ends of the cutting elements, which may be configured to retain each of the cutting elements apart from another during balloon inflation, where the radially intermediate expandable section: s) can comprise a closed cell structure configured to expand and contract, while keeping cutting elements from twisting or entangling; t) can comprise a plurality of connection points to cutting elements; u) the cutting elements are configured in a closed loop arrangement, wherein: v) the cutting elements are arranged as parallel struts such that the closed loop structure arranged along the axial direction of the struts; o the cutting elements are arranged so as to prevent entanglement of the struts during balloon inflation; w) the cutting elements are connected at a distal end to the tip portion; x) the tip portion is configured with a closed loop structure; and/or y) a proximal end of the cutting elements are connected to the proximal collar.

In some embodiments, a stenosed vessel treatment system is provided and includes the device according to any of the disclosed embodiments (e.g., any of the embodiments noted above), a balloon catheter, and/or a tracking guidewire. The scoring device of any of the embodiments of the invention is provided as an add-on device to be used with a balloon catheter. Alternatively, the scoring device disclosed may be provided as part of (e.g., pre-assembled with) a balloon catheter or other similar stenosed vessel treatment device. In use, the scoring device of any of the embodiments of the invention may be assembled on the balloon catheter and then brought to the treatment site in the patient's body, such as over a guidewire. Alternatively, the scoring device of any of the embodiments of the invention may be first brought to the treatment site in the patient's body, such as over a guidewire, and then afterwards, the balloon catheter may be brought to the treatment site in the patient's body, such as over the same guidewire, wherein the balloon catheter couples with the scoring device in-situ at the treatment site.

The above noted embodiments may include one and or another of the following features, structure, function, steps, and/or clarifications which, together in various combinations, provide yet further embodiments of the disclosure: a) the balloon catheter apparatus comprises a shaft, a tip section, and an expandable balloon configured to expand outwardly when inflated and retract inwardly when deflated; and b) an inflator, which may be connected to the balloon catheter apparatus via a luer located on a proximal end of the catheter shaft.

A non-claimed lesion scoring method is also described and includes providing a lesion scoring system according to any of the disclosed embodiments (e.g., the system embodiments described above), inserting the tracking guidewire into the vessel, back-loading the tracking guidewire into the distal tip of the device, advancing the device over the tracking guidewire until the distal end of the device is adjacent a lesion of interest, advancing the balloon catheter over the tracking guidewire until the distal end of the balloon catheter reaches a proximal end of the distal tip of the device, and inflating the balloon so as to force at least one of the plurality of wires against one or more corresponding sides of a lesion in the vessel. Such a method may further include one or more of deflating the balloon, and withdrawing the balloon catheter and the device, either independently or together.

These and other embodiments and features will become even more evident in view of the detailed description which follows and included drawings, a brief description of which is provided below.

Unless otherwise defined, all technical and scientific terms used herein have the same meaning as commonly understood by one of ordinary skill in the art to which embodiments of this disclosure belongs.

### BRIEF DESCRIPTION OF THE FIGURES

Figure 1 is an illustration of a prior art balloon catheter crossing a lesion within a blood vessel.
Figure 2 is an illustration of a prior art process for expanding a balloon in a lesion within a blood vessel.
Figure 3 is a schematic representation of a guidewire to percutaneously reach areas of interest within the vascular system.
Figure 4 shows a wire cutting assembly, according to some embodiments, as it is placed over a guidewire.
Figure 5 is a schematic representation of a percutaneous transluminal coronary angioplasty ("PTCA") balloon catheter inserted over the guidewire after a wire cutting assembly, according to some embodiments.
Figure 6 is a schematic representation of a wire cutting assembly and a balloon catheter across a lesion in a blood vessel, according to some embodiments.
Figure 7 is a schematic representation of a balloon as it is inflated within a lesion in a blood vessel, according to some embodiments of the present disclosure.
Figure 8 is a schematic representation of a wire cutting assembly, according to some embodiments, where two cutting wires are positioned in parallel and extend towards a proximal end of wire cutting assembly.
Figure 9 is an illustration of a wire cutting assembly, according to some embodiments, where two (2) wires are arranged in a helical configuration around a central axis defined by an inner lumen of a tip .
Figure 10 is an illustration of a wire cutting assembly, according to some embodiments, where three (3) wires are included and form a helical section.
Figure 11 is a schematic representation of a standard balloon catheter and a wire cutting assembly positioned over a guide wire, according to some embodiments.
Figure 12 is a schematic representation of a scoring device for configured to be arranged on an in-line balloon catheter according to some embodiments.
Figure 13 illustrates a tip-portion/section of the scoring element of Figure 12, according to some embodiments.
Figure 14A illustrates a portion of an add-on scoring device showing a proximal portion having a snap element for removable affixation to a balloon catheter, according to some embodiments.
Figure 14B illustrates a cross-section of an attachment element, according to some embodiments.
Figure 15 is a schematic representation of a standard prior art catheter balloon.
Figure 16 illustrates a scoring device arranged with a balloon catheter, according to some embodiments.
Figure 17 is an enlarged view of an attachment element for a scoring device for use with removably affixing the scoring device to a balloon catheter, according to some embodiments.
Figures 18 and 18A-18C illustrate other attachment elements placed over a balloon catheter shaft, for example, according to some embodiments.
Figure 19 is an enlarged view of a distal end of a scoring device placed over a balloon catheter, according to some embodiments.
Figure 20 is a schematic representation of a scoring device, according to some embodiments, having longitudinal cutting elements.
Figure 21 is an enlarged view of a closed expandable structure of a scoring device, according to some embodiments.
Figure 22 is a schematic representation of a flattened (for example) metal-cut construct of a closed loop device, according to some embodiments, used to manufacture a scoring device according to some embodiments.
Figure 23 is another schematic representation of the device of Figure 22, according to some embodiments.
Figures 24A-D are schematic representations of exemplary embodiments of a scoring device, illustrating a closed loop diamond like scoring device configuration (Figure 24A, C), a parallel scoring device configuration (Figure 24B), as well as a hex-shaped scoring device configuration (Figure 24D).
Figure 25A-B are schematic representations of a scoring device, according to some embodiments, having one or more bent-wire configurations along the length of the scoring devices, Figs. 26A-26F are schematic representations of how to load the universal Cutting Add-on Device (CAD) onto a standard balloon catheter, and Fig. 27A-27B are schematic representations of another attachment structure for coupling any of the add-on cutting/scoring devices of the invention proximally to the balloon catheter shaft.

### DETAILED DESCRIPTION OF SOME OF THE EMBODIMENTS

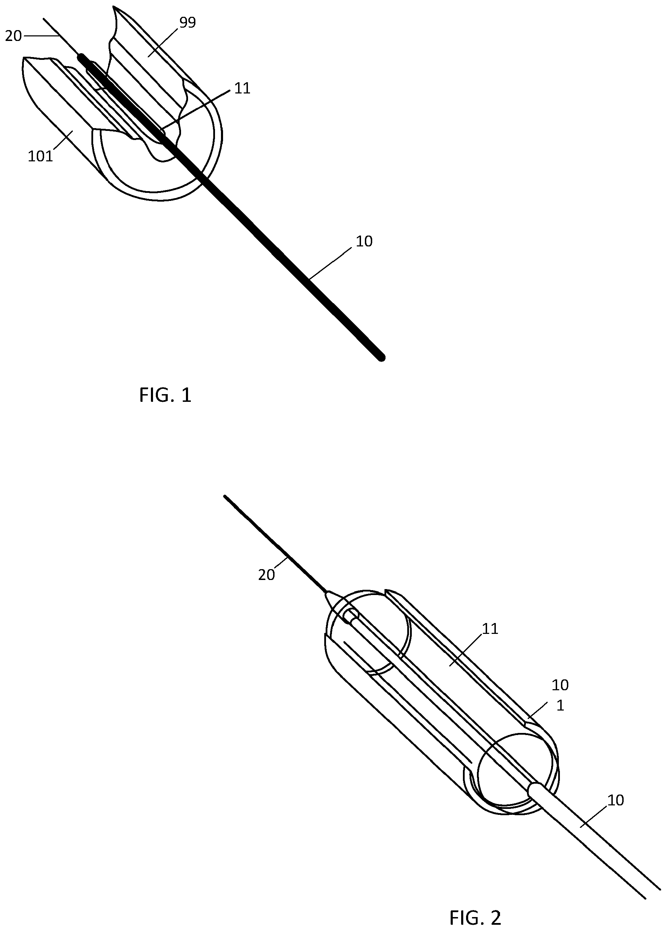

Figures 1 and 2 illustrate a standard balloon and guidewire system for expanding a blood vessel. Accordingly, Figure 1 is an illustration of a standard PTCA balloon catheter 10 crossing a lesion 99 within a blood vessel 101. A guidewire 20 is used to cross the lesion first, followed by placing the balloon catheter 10 over the guidewire 20, until a distal end of the balloon 11 crosses the lesion. As shown in Figure 2, which is an illustration of the ballooning process for a lesion within in a blood vessel 101, once the balloon 11 crosses the lesion, the balloon can be inflated resulting in the compression of the lesion outward radially, which can restore flow within the blood vessel.

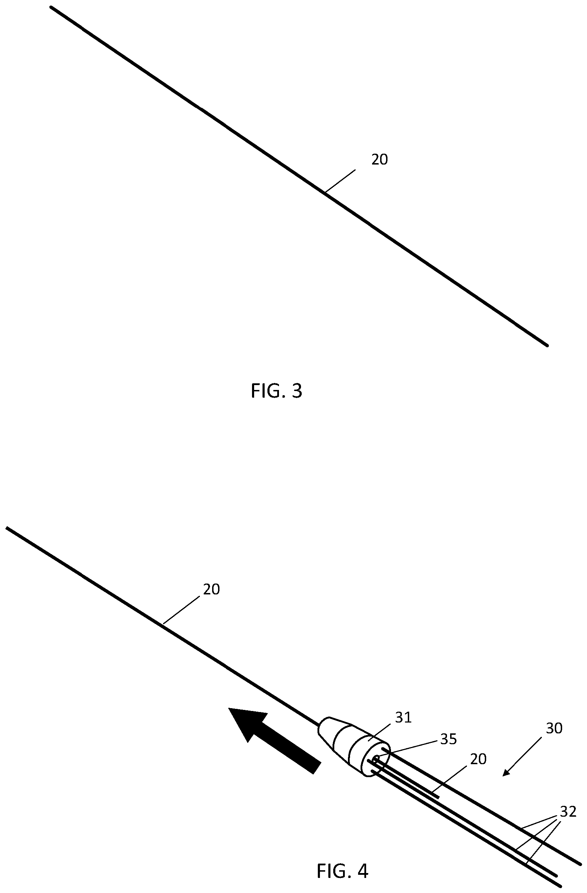

Figure 3 is a schematic representation of a guidewire 20 which may be used in some embodiments of the present disclosure, to percutaneously reach areas of interest within the vascular system. Overtop the guidewire 20, in the direction of the arrow (for example), a scoring device 30 (also referred to herein as a wire cutting assembly 30) is provided, as shown in Figure 4. As shown, the scoring device 30 may comprise a scoring structure 32, such as scoring wires, and a tip 31. One end of the scoring device includes an opening 33 through which the balloon is extractable after the scoring device is assembled on the catheter shaft. Tip 31 may be formed with a guidewire aperture 35. Tip 31 may be solid or may be formed with a hollow portion (central to, or off-center from, the longitudinal axis of the tip 31). Tip 31 serves as abutment structure against which the balloon 11 can abut. The distalmost end of the balloon 11 can abut against the tip, or alternatively, the distalmost end can be narrow enough to pass through the guidewire aperture 35 but the rest of the balloon is stopped by the tip from passing further through past the tip. For a solid tip 31, the balloon is arrested by the proximal face of tip 31; for a hollow tip, the balloon enters the hollow portion and is arrested by the distal inner wall of the hollow tip.

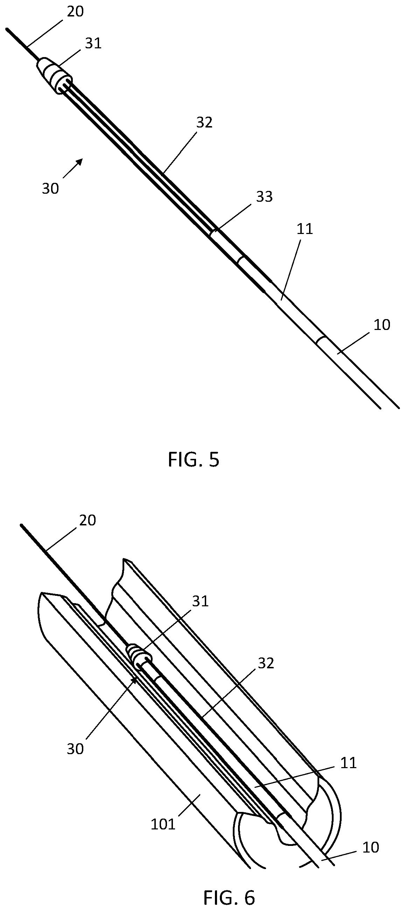

As shown in Figure 5, which is a schematic representation of a balloon catheter 10 inserted over the guidewire 20 after the wire cutting assembly 30 is inserted there over. In some embodiments, the result of the combination of the cutting assembly 30 in combination/addition to the balloon catheter 10 is that once the balloon 11 is inflated within the lesion, the wire(s) 32 of the scoring structure, which are located on the outer contour of the balloon, expand outwards and score the lesion. Figure 6 shows a schematic representation of the cutting assembly 30 and the balloon catheter 10 across a lesion in a blood vessel 101. Both the assembly and the balloon are placed over guidewire 20.

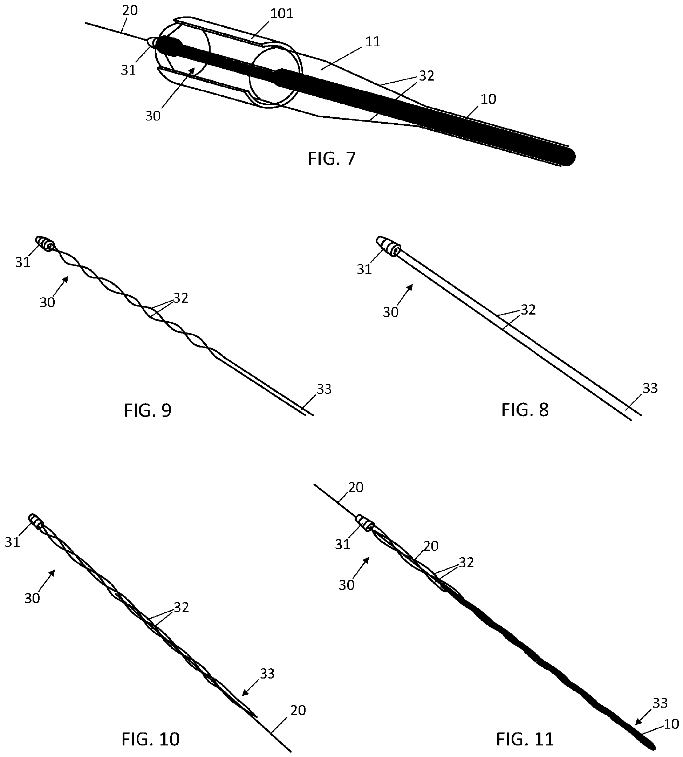

Accordingly, shown in Figure 7 is a schematic representation of the balloon 11 as it is inflated within the lesion in blood vessel 101. The cutting wires 32 of the cutting assembly 30 are pressed between the balloon 11 and the inner wall of the lesion during balloon inflation, causing scoring of the lesion structure. In this orientation, the wires 32 can extend from the tip 31 generally parallel to and radially around the guidewire 20. As illustrated in FIG. 6, the balloon catheter 10 can be fitted over the guide wire 20 and advanced along the guide wire 20 such that the tip of the balloon catheter 10 is positioned adjacent to the tip 31 of the wire cutting assembly 30. In this position, at least a portion of the balloon catheter 10 is received between the radially arranged wires 32 such that at least a position of the wires 32 can extend over the balloon 11 of the balloon catheter 10. Inflation of the balloon 11 presses the cutting wires 32 of the cutting assembly 30 between the balloon 11 and the inner wall of the lesion causing scoring of the lesion structure.

Figure 8 is a schematic representation of a cutting assembly 30, according to some embodiments, which includes two cutting wires 32 positioned in parallel, for example, and extend towards the proximal end of the cutting assembly 30 (proximal, i.e., towards the user/surgeon). The cutting wires can be positioned at or near 180 degrees to one another, for example, but can also be positioned at any radial angle relative to one another. The invention is not limited to this arrangement and the wires can be positioned in other orientations. In some embodiments, one of the cutting wires 32 can includes a larger cross-sectional diameter than the other. In such embodiments, such a configuration enables pushability of the cutting assembly, that is, the wire assembly can be easier to push along the guidewire within the vessel, so that the operator can more easily control the axial position of the device.

Figure 9 is an illustration of the cutting assembly 30, according to some embodiments, illustrating a cutting assembly comprising at least two (2) wires which can be configured to form a helical configuration around a central longitudinal axis of the tip 31. The length of the helical section can be any length, and, in some embodiments, can be as long as the device itself (e.g., the helical section can extend from the distal tip 31 all the way back to the proximal end of the device). In some embodiments, the helical section can be tailored to a specific length of any standard balloon. The helical portion can be pre-formed by the manufacturer, or can be formed by the surgeon and the like, and can be modified if needed. There can be a combination of a helical and non-helical section (such as a parallel wire section). The helical section can have any number of twists and shapes and distances between twists.

Figure 10 is an illustration of the cutting assembly 30, according to some embodiments, illustrating a cutting assembly comprising at least three (3) wires which can be configured to form a helical section, which can also be joined by any method known in the art so as to form a connection from which one (1) or more cutting wires 32 extend towards the proximal end of the wire cutting assembly 30. The wire or wires allow for axial pushability within the vasculature; that is, the cutting assembly is easy to move axially along the guidewire within the blood vessel. As stated above, the wires used to construct the helical section can be of different diameters, and can be positioned at different radial angles to one another. It is noted that any of the rigidity of the wires of any of the embodiments of the invention may be designed for just pulling, but not pushing, or alternatively for pushing, but not pulling, or alternatively for pushing and pulling. For example, for pulling, the wire can be more flexible and/or thinner; for pushing, the wire may be more rigid and/or thicker. Although in most applications the tip 31 will go first over the guide wire 20, optionally, as seen in Fig. 10, the guide wire 20 can be inserted through opening 33.

Figure 11 is a schematic representation of a balloon catheter 10, and a cutting assembly 30 over a guide wire 20, according to some embodiments, where the cutting assembly 30 includes a plurality of cutting wires 32, which can be configured to form a helical pattern at the 11 distal end of the cutting assembly 30, where, in some embodiments, one wire extends in a direction back towards the proximal end of the device 30. The wires can extend partially to the proximal end; alternatively, extend to be flush with the proximal end; or alternatively, extend proximally beyond the proximal end.

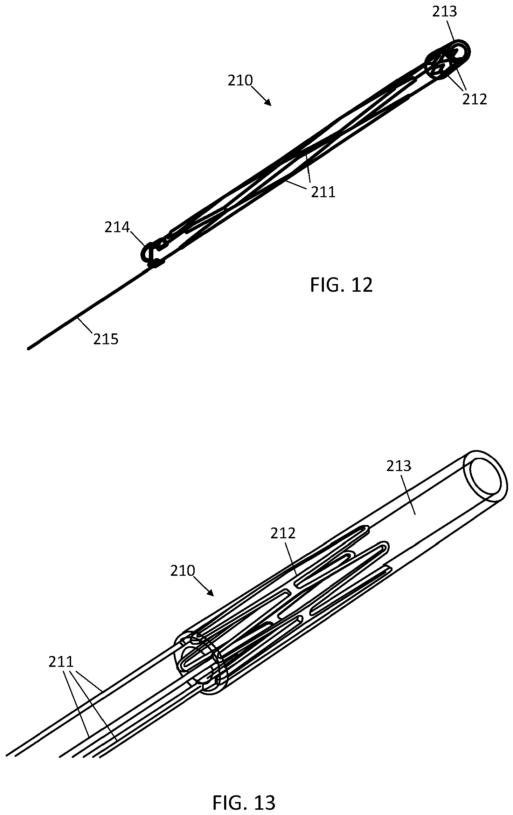

Figures 12-25B represent further embodiments of the present disclosure.

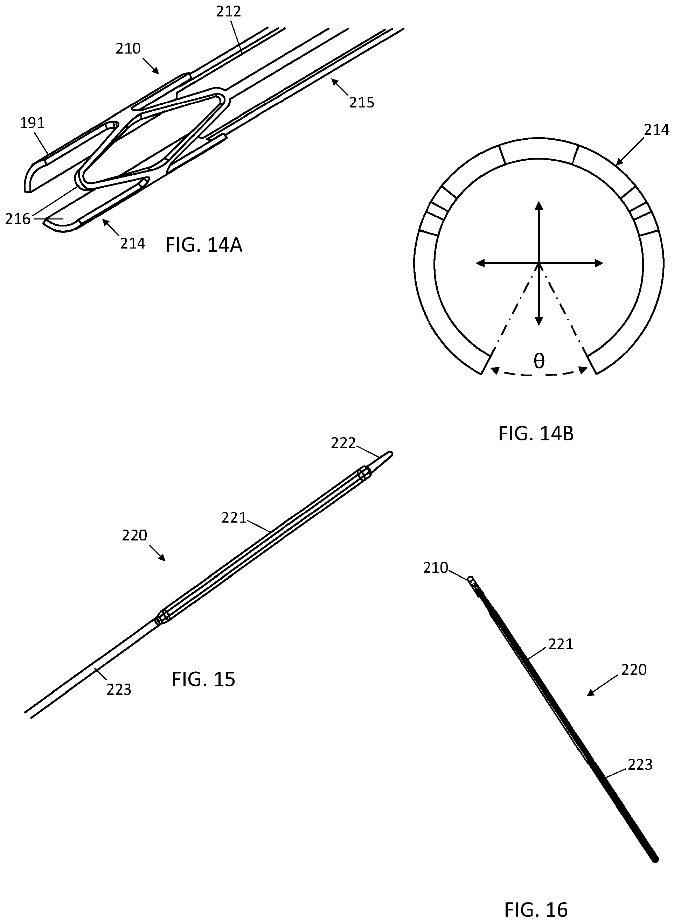

Figure 12 is a schematic representation of an add-on scoring device, configured for arrangement in-line with a balloon catheter (not shown in figure). The add-on scoring device 210 has a main body having one or more (preferably two or more) expandable and contractible cutting elements 211 to allow add-on device 210 to expand and retract in the radial direction with the inflation/deflation of the balloon. In general, the scoring capability of the device increases with an increased number of cutting elements 211. Add-on scoring device 210 also can include a tip section which allows a certain degree of radial expansion (e.g., without limitation, up to five times the initial diameter) so as to accept a tip of the balloon catheter (not shown). The tip section is shown with an over-mold structure 213 (e.g., a cover) which can be made of a polymeric material which is flexible and soft. The cover can completely or partially cover the tip section. The tip section can retain the ability to expand and retract in the radial direction. Add-on device 210 also can include an attachment element, or snap-collar 214, which may be proximally spaced (e.g., relative to the tip which is distal) which may be constructed of a generally cylindrical band which includes an open portion (shown by theta in Fig. 14B) which may be between 10 degrees and 180 degrees, so as to allow the snap-collar to be attached to a shaft of a balloon catheter (not shown), without the risk of detaching/coming-free, yet still retain the ability to freely slide over the shaft in the axial direction thereof. A wire control element 215 (e.g., tether wire) may be connected to collar 214 or to the tip or any other portion of the device, and extend proximally such that its proximal end is accessible to the operator. The wire control element may be used to extract the scoring device from the body lumen (such as by pulling), and/or may be used to maneuver the scoring device while in the body lumen (such as by pulling and/or pushing and/or rotation). The wire control element may of any length: long enough to extend out of the patient's body for grasping by the surgeon, or can be short and not extend out of the body and grasped by a tool. It can be fitted within a deployment catheter.

Figure 13 shows another version of the tip section of the add-on scoring device. As shown, the over-mold tip section 213 can be seen over an expandable laser-cut element 212. Accordingly, when a radial force is applied to the tip section, the cells 212 (e.g., which may be laser cut) can expand outwardly in a radial direction and retract when said force is removed. The over-mold tip section 213 may be constructed of a material which is softer than the expandable element 212, such as but not limited to, an elastomeric material, e.g., silicone, latex or synthetic rubber. This feature may help prevent the device from damaging body lumens; the soft tip abuts against the inner wall of the body lumen, thereby protecting the body lumen from the harder expandable element. The tip may have a bullet shape, a tapering shape, an arrow shape or other suitable shapes.

Figure 14A shows a portion of add-on scoring device 210, specifically, the proximal snap 214 which can firmly snap on to a wide range of balloon catheter shafts (not shown) due to an expandable section 216 which can widen when the balloon catheter is axially inserted; the thickness of the balloon catheter imparts a radial expanding force on the expandable section 216. Proximal snap 214 can fit over the balloon catheter shaft so as to prevent dislodgement of add-on scoring device 210 and yet allows movement in the axial direction of the shaft (due to a loose fit and/or low friction), in essence, allowing add-on scoring device 210 to shorten when its diameter expands with the inflation of the balloon and retracts when the balloon is deflated. The expandable section 216 of the proximal snap (also referred to as attachment structure) 214 may be formed as a resilient wire-frame structure with circumferentially-curved proximal ends 191 so that proximal snap 214 can securely fit over the circumferential outer contour of a wide diameter range of balloon catheter shafts. The snap 214 expands or contracts in size to match the particular diameter of the catheter shaft.

Figure 14B is a representation of a cross-section of a proximal snap 214 which shows the degree of opening of the snap. The opening is represented by the angle Θ (theta), which, according to some embodiments, can range between about 1 to 179.9 degrees.

Figure 15 is a schematic representation of a standard catheter balloon 220, as is known in the art, which typically consists of a shaft 223, a tip section 222 (which is typically soft and supple) and an expandable balloon 221 configured to expand outwardly when inflated and retracts when deflated. Inflation and deflation are typically controlled by an inflator which is connected to the balloon via a luer connector located (for example) on a proximal end of the catheter shaft.

Figure 16 is an illustration of add-on scoring device 210 configured as a sleeve that fits over a balloon catheter 220/ Both scoring device 210 and catheter 220 can be advanced and retracted over a guidewire.

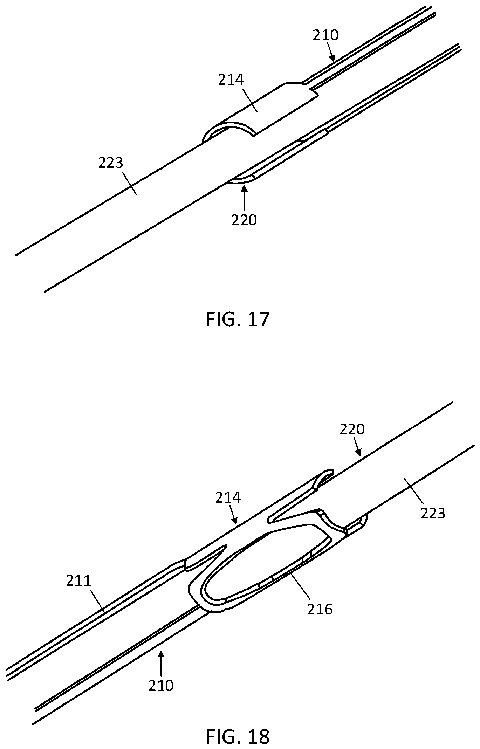

Figure 17 is an enlarged view of a proximal snap 214 of an add-on scoring device 210 placed over balloon catheter 220. Here, the proximal snap 214 is shown as it fits over balloon catheter shaft 223, with the opening of the snap (which may also be referred to as a cuff or affixation element) configured to allow placement over the catheter shaft allowing for accommodation of the shaft while preventing dislodgement.

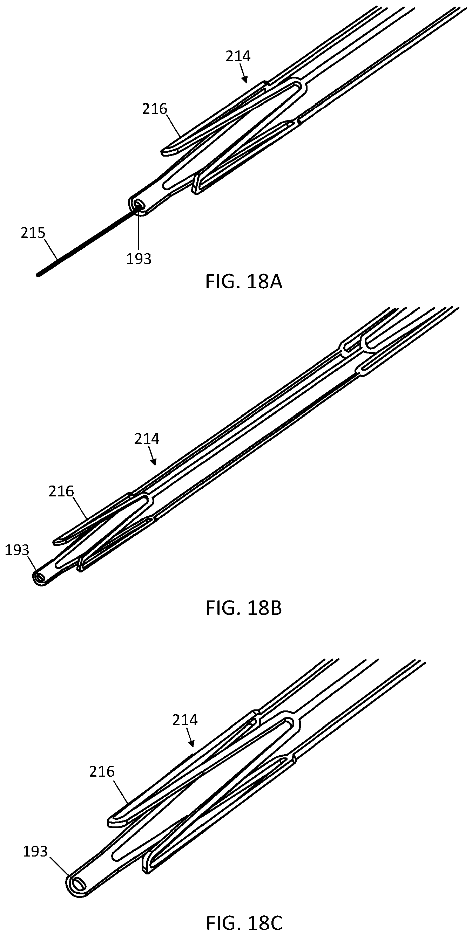

Figure 18 is yet another view of the proximal snap 214 placed over balloon catheter shaft 223, wherein expanding section 216 of the snap cuff 214 is shown in greater detail. Expanding section 216 allows the snap cuff 214 to increase in diameter when a large balloon catheter shaft is encountered. This functionality enables the snap cuff 214 to expand (due to the structure of expanding section 216) and move relative to the catheter shaft in the axial direction thereof. Other expandable snap cuffs are shown in Figures 18A-18C. Here again, the expandable section 216 of the attachment structure 214 may include resilient wire-frame structure that securely fits over the circumferential outer contour of a wide diameter range of balloon catheter shafts. The snap 214 expands or contracts in size to match the particular diameter of the catheter shaft. A mounting hole 193 is formed at the proximal end of attachment structure 214 for attaching thereto a tether wire 215, such as by tying, bonding, welding or any other joining method. As seen in Fig. 18C, the expandable structure 216 may include arms or tongues of different lengths; for example, the arm with the mounting hole 193 may be longer than the other arms.

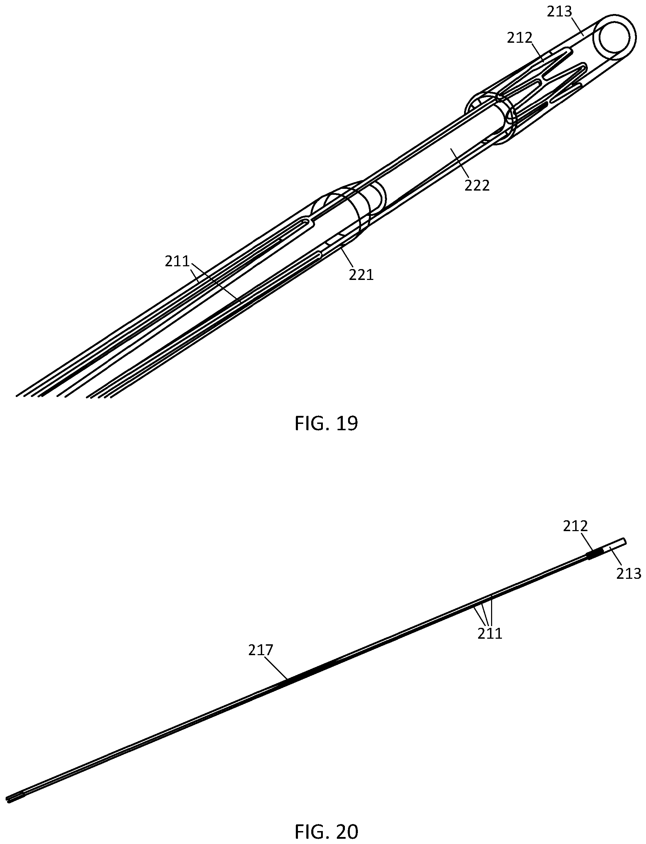

Figure 19 is an enlarged view of the distal end of add-on scoring device 210 placed over a balloon catheter 220, with the tip 222 of balloon catheter 220 being arranged proximal to the tip 212 of add-on scoring device 210. In some embodiments, since tip 212 is configured to expand in the radial direction, it can accommodate at least a portion of the tip 222 of the balloon catheter 220, holding it in place without the risk of relative movement during balloon inflation. As explained above, tip 212 is abutment structure that prevents the balloon from being pushed through tip 212. Cutting elements 211 are shown over the balloon section 221, and can be configured to move in the radial direction (expand and contract) with the inflation/deflation cycle of the balloon 221.

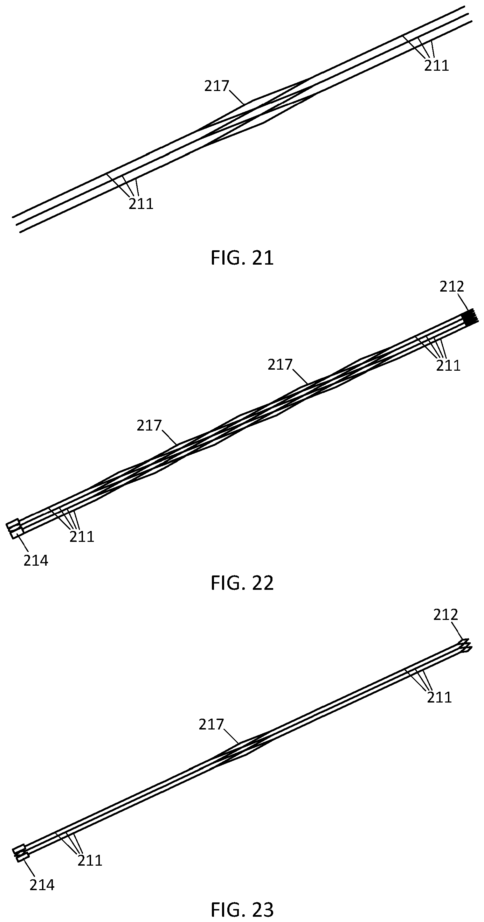

Figure 20 illustrates yet other embodiments of the add-on scoring device 210 of the present disclosure, illustrating longitudinal cutting elements 211. Here, a radially expandable section 217 is shown which is located along the length of cutting elements 211. The expandable section is designed to keep the cutting elements 211 apart from each other during balloon inflation so as to avoid a risk of possible entanglement of the cutting elements when the balloon (not shown) is inflated (due to vessel tortuosity). Entanglement of cutting elements during inflation can damage the add-on scoring device 210, as well as the vasculature. Radial expansion section aids in avoiding entanglement by providing a closed cell structure which, while expandable, functions to connect to the cutting elements 211 at either end (of the cell structure) such that the cutting elements cannot twist freely. To this end, Figure 21 illustrates a close up of a closed, expandable structure showing connection points to cutting elements 211. Without limitation, expandable section 217 may expand over a range of 2-15 mm, or alternatively 3-10 mm or other ranges, depending on the need. The wires (cutting elements 211) are attached to the proximal and distal ends of expandable section 217. The expandable section 217 expands radially outwards upon inflation of the balloon. This radial expansion spreads the wires that are attached to the expandable section 217 radially outwards, thereby preventing the wires from getting tangled with each other.

Figure 22 is a schematic representation of a flattened, metal-cut construct, of a closed loop structure which can be used to manufacture an add-on scoring device 210, according to some embodiments. To this end, the cutting element 211 is designed as a closed loop, with the cutting element 211 being connected at the distal end to the tip, which is also a separate closed loop design. At the proximal end, the cutting element section 211 is connected to a proximal snap cuff 214. Figure 23 is a schematic representation of the flattened metal cut construct of the closed loop embodiment of Figure 22, illustrating the design of the cutting element 211. Here, the cutting element 211 is configured as parallel struts with a closed loop structure along the axial direction of the struts, which prevents entanglement of the struts during balloon inflation.

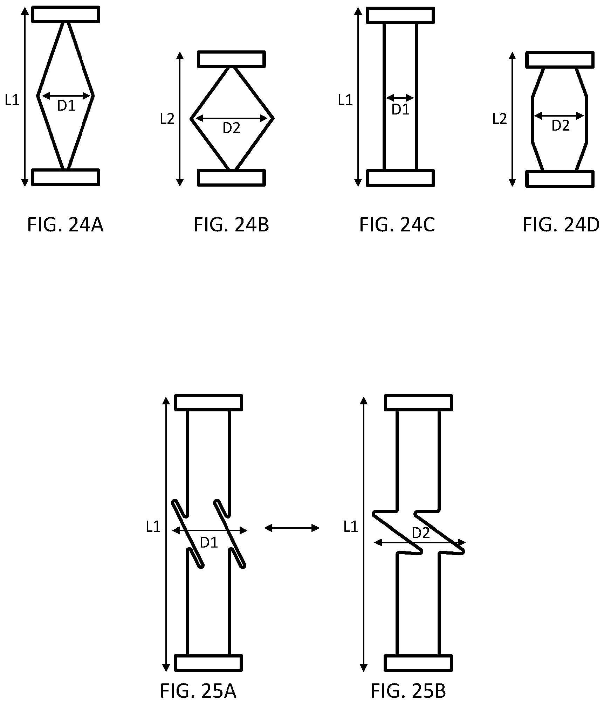

Figure 24A-D is a schematic representation of a flattened add-on scoring device, according to some embodiments, where the add-on scoring device is constructed of a closed loop diamond like cutting elements 211 configuration, Figure 24A and 24C, parallel cutting elements 211 configuration, Figure 24D, and a hex-shaped configuration, Figure 24B. The scoring add-on device is fitted over a standard balloon catheter (and advanced with the balloon to the lesion site, as with other disclosed embodiments). Before the balloon is inflated, the add-on scoring device has a length of L1 and a diameter which corresponds to D1. When the balloon is inflated, the diameter of the scoring add-on device increases with the diameter of the balloon and its length is reduced to L2 and the diameter increases to a value corresponding with D2. The process is reversed when the balloon is deflated.

Figure 25A-B is a schematic representation of a flattened add-on scoring device which has one or more scoring elements 211 with bent portions 311 (according to a number of possible bending configurations - illustrations of which are merely examples) along the length of the scoring elements 211. The bent portion in Fig. 25A has a tight, narrow, italic S-shaped formation, whereas the bent portion in Fig. 25B has a more spread out S-shaped formation. They are folded over each other so they can increase the effective length of the scoring elements. These bent-portions allow the scoring elements 211 to lengthen during balloon inflation, without foreshortening the length L1 of the scoring add-on device. While the diameter of the scoring add-on device changes from a value corresponding to D uninflated balloon state to D inflated balloon state, the length L1 stays constant. This is very helpful in tortuous anatomies such as an arteriovenous fistula.

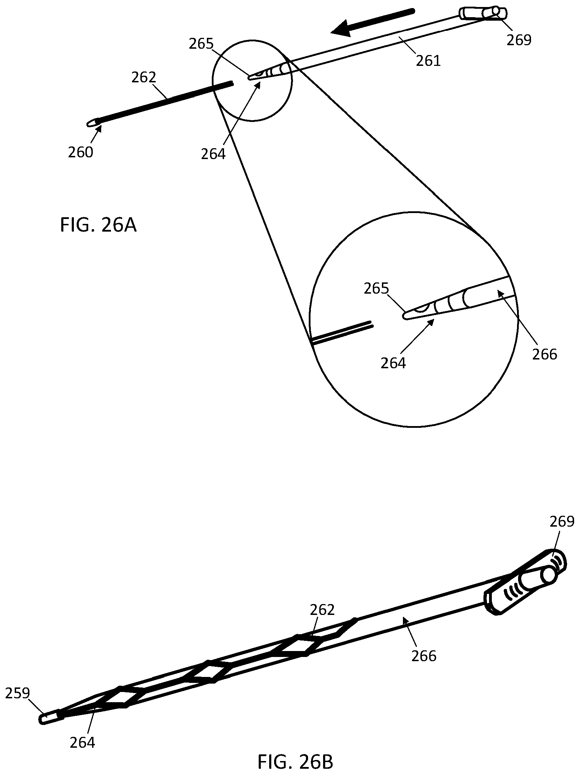

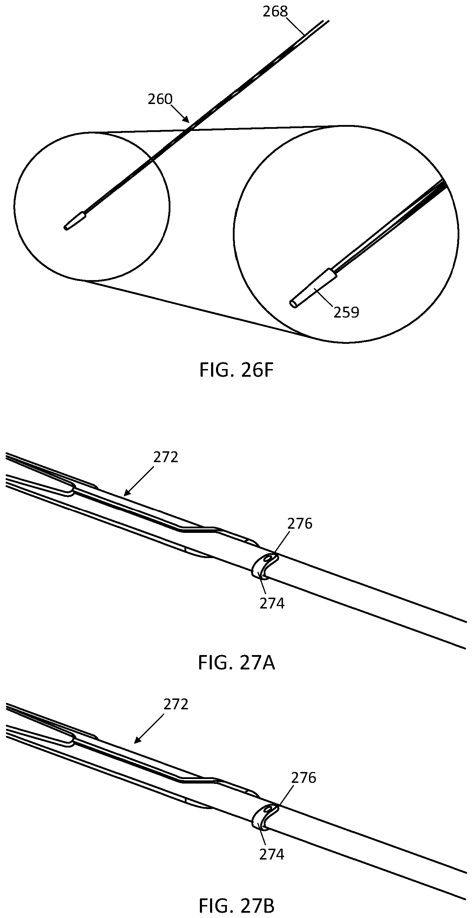

Reference is now made to Figs. 26A-26F, which describe how to load the universal cutting add-on device 260 onto a standard balloon catheter. The add-on device 260 is designed to fit onto any PTCA catheter. It consists of the distal cutting element (scoring structure) 262 and may optionally include a tether wire which extends proximally (not shown here for simplicity but shown in other embodiments above). The add-on device 260 is designed to fit over the balloon section of the balloon catheter so that the cutting element (scoring structure) 262 is located over the balloon. A mandrel may optionally be used to facilitate placing the add-on device 260 over the balloon section. The description follows with the use of a dilator system, but this is optional and the scoring device according to the invention can be used without the dilator system. (It is noted that the mandrel is a conical tool that dilates the add-on device as described for the dilator system.)

Step 1 (with reference to Figs. 26A-B): A concentric sheath and dilator system 261 includes a dilator 264, having a conical tip 265, with a sheath 266 placed over the shaft of the dilator. The tip 265 protrudes distally past sheath 266. The dilator 264 is first inserted into the proximal end of the scoring structure 262 of add-on device 260, opening the add-on device 260 radially while it is advanced until the tip of a dilator 264 presses against the tip 259 (Fig. 26B) of the add-on device 260. At this point the add-on device 260 snugly fits over sheath 266.

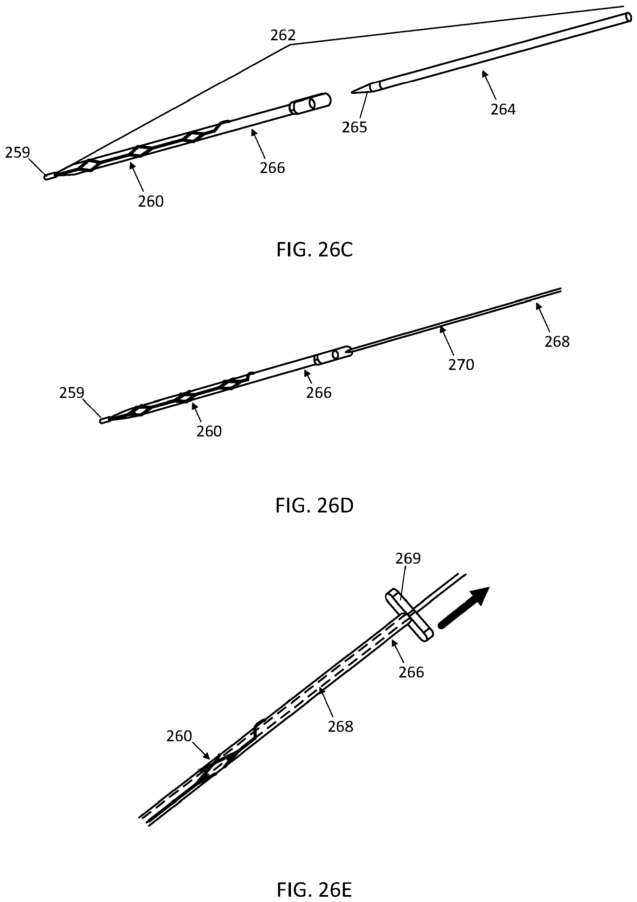

Step 2 (with reference to Fig. 26C): The dilator 264 is removed from the sheath 266 by grasping a proximal handle 269 of dilator 264 and pulling the dilator out in the proximal direction. Sheath 266 has sufficient stiffness to retain its shape even after the dilator 264 is removed and separated away from the sheath.

Step 3 (with reference to Fig. 26D): At this point, a balloon catheter 268 with a balloon 270 is inserted into the sheath 266 and advanced until the tip of the catheter 268 presses against the tip 259 of the add-on device 260.

Step 4 (with reference to Figs. 26E-F): The sheath 266 is pulled back and removed (optionally, the sheath may be a peel-away sheath). The add-on device 260 then contracts radially inward and fits snugly over the balloon section of the balloon catheter.

Fig. 27A-27B illustrate another attachment structure 272 for coupling any of the add-on cutting/scoring devices of the invention proximally to the balloon catheter shaft. The attachment structure 272 includes a pigtail configuration 274 that wraps around the catheter shaft and which can be connected by gently twisting the proximal end of the add-on cutting device around the shaft. A hole 276 at the base of the pigtail element 274 can be used to connect a tether wire (not shown) which can be stiff, supple or flexible. The scoring wires may be joined to the attachment structure 272 (at any suitable portion distanced from the pigtail element 274) by any suitable joining method, such as but not limited to, welding, bonding and others. Axial pulling of the pigtail element causes the pigtail element to contract and squeeze tighter against the catheter shaft.

In summary, in any of the above description, there is provided a catheter system including a balloon catheter including a catheter shaft and an expandable and contractible balloon mounted on the catheter shaft, and a scoring device including scoring structure configured to score a material inside a body lumen, the scoring device being assembled over the balloon on the catheter shaft and including a first end and a second end, wherein the first end includes an opening through which the balloon is extractable after the scoring device is assembled on the catheter shaft. The balloon may be moved through the opening before, during and after in-situ delivery of the catheter system to the body lumen. The first end may be the proximal end and the second end may be the distal end. However, in other options and delivery configurations, the first end may be the distal end and the second end may be the proximal end. The scoring device can adapt to any size balloon catheter as an add-on device (or optionally pre-assembled to the balloon catheter).

The second end may include abutment structure through which at least a portion of the balloon cannot pass.

The first and/or the second end may be slidingly mounted on the catheter shaft.

The scoring structure may expand away from the catheter shaft upon expansion of the balloon.

The scoring structure may include at least one wire extending between the first and second ends.

The scoring device may include a tip which is non-injurious to an interior of the body lumen. The tip may be or may include the abutment structure through which at least a portion of the balloon cannot pass.

The scoring device may be assembled on the catheter shaft with a fastening element which is expandable outwards from the catheter shaft and contractible inwards towards the catheter shaft.

The scoring device may be assembled on the catheter shaft with a fastening element which wraps around at least a portion of an outer periphery of the catheter shaft.

The scoring device may include a proximally extending control wire.

The scoring device may include a radially expandable section adjacent the scoring structure.

In any of the above description, there is provided a method of using a catheter system including the step of introducing a scoring device and a balloon catheter to a site in a body lumen, the balloon catheter including a catheter shaft and an expandable and contractible balloon mounted on the catheter shaft, and the scoring device including scoring structure configured to score a material inside the body lumen, the scoring device being assembled over the balloon on the catheter shaft and including a first end and a second end, wherein the first end includes an opening through which the balloon is extractable after the scoring device is assembled on the catheter shaft, and expanding the balloon and manipulating the scoring device to score the material inside the body lumen.

The scoring device may be assembled over the balloon on the catheter shaft before introduction of the scoring device and the balloon catheter to the site in the body lumen.

The scoring device may be first introduced to the site in the body lumen and then afterwards the balloon catheter may be introduced to the body lumen, and at the site, the balloon may be placed inwards of the scoring device.

The balloon may be extracted out of the scoring device through the opening after manipulating the scoring device to score the material inside the body lumen.

The balloon catheter may be selected from a plurality of balloon catheters that have different size shaft peripheries, and the scoring device may be assembled on the catheter shaft with a fastening element which is expandable outwards from the catheter shaft and contractible inwards towards the catheter shaft such that the scoring device is mountable on any of the balloon catheters.

While various inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the present disclosure. More generally, those skilled in the art will readily appreciate that all parameters, dimensions, materials, and configurations described herein are meant to be an example and that the actual parameters, dimensions, materials, and/or configurations will depend upon the specific application or applications for which the disclosed teachings is/are used. Those skilled in the art will recognize, or be able to ascertain using no more than routine experimentation, many equivalents to the specific embodiments described herein. It is, therefore, to be understood that the foregoing embodiments are presented by way of example only. Embodiments of the present disclosure are directed to each individual feature, system, article, material, and/or kit, described herein. In addition, any combination of two or more such features, systems, articles, materials, and/or kits, if such features, systems, articles, materials, and/or kits are not mutually inconsistent, is included within the scope of the present disclosure. Some embodiments may be distinguishable from the prior art for specifically lacking one or more features/elements/functionality.

## Claims

1. A scoring device (30; 210) comprising a scoring structure (32; 211) configured to score a material inside a body lumen (101), said scoring device comprising a first end and a second end,
wherein said first end comprises an opening (33),
wherein said opening is configured for a balloon (11; 221) mounted on a catheter shaft to pass therethrough into said scoring structure and for the balloon to be completely extracted out of said scoring device, and
**characterized in that**
the first end further comprises a fastening element (214; 272) configured to attach to the balloon catheter proximally spaced to the balloon.

2. The scoring device according to claim 1, wherein said second end comprises a guidewire aperture (193) or an abutment structure (212) configured to block the balloon from completely passing therethrough.

3. A catheter system comprising:
a balloon catheter (10) comprising a catheter shaft and an expandable and contractible balloon (11) mounted on said catheter shaft; and
a scoring device (30) as claimed in claim 1 or claim 2, , said scoring device being assembled over said balloon (11) on said catheter shaft, wherein said balloon is extractable through the opening (33) after said scoring device is assembled on said catheter shaft.

4. The catheter system according to claim 3, wherein said balloon (11) is movable through said opening (33) before, during and after in-situ delivery of said catheter system to said body lumen.

5. The catheter system according to claim 3, wherein said second end comprises abutment structure through which at least a portion of said balloon cannot pass.

6. The catheter system according to claim 3, wherein at least one of said first and second ends is slidingly mounted on said catheter shaft.

7. The catheter system according to claim 3, wherein said scoring structure (32) expands away from said catheter shaft upon expansion of said balloon.

8. The catheter system according to claim 3, wherein said scoring structure (32) comprises at least one wire extending between said first and second ends.

9. The catheter system according to claim 3, wherein said scoring device (30) comprises a tip which is non-injurious to an interior of said body lumen.

10. The catheter system according to claim 9, wherein said tip comprises an abutment structure through which at least a portion of said balloon cannot pass.

11. The catheter system according to claim 3, wherein said scoring device (30) is assembled on said catheter shaft with the fastening element and wherein the fastening element is expandable outwards from said catheter shaft and contractible inwards towards said catheter shaft.

12. The catheter system according to claim 3, wherein said scoring device (30) is assembled on said catheter shaft with the fastening element and wherein the fastening element wraps around at least a portion of an outer periphery of said catheter shaft.

13. The catheter system according to claim 3, wherein said scoring device (30) comprises a proximally extending control wire (215).

14. The catheter system according to claim 3, wherein said scoring device (30) comprises a radially expandable section (217) adjacent said scoring structure (32).

## Patentansprüche

1. Eine Einkerbungsvorrichtung (30; 210), die eine Einkerbungsstruktur (32; 211) beinhaltet, die so konfiguriert ist, dass sie ein Material innerhalb eines Körperlumens (101) einkerbt, wobei die Einkerbungsvorrichtung ein erstes Ende und ein zweites Ende beinhaltet,
wobei das erste Ende einen Durchbruch (33) beinhaltet,
wobei der Durchbruch so konfiguriert ist, dass ein an einem Katheterschaft montierter Ballon (11; 221) durch ihn hindurch in die Einkerbungsstruktur eintreten kann und der Ballon vollständig aus der Einkerbungsvorrichtung herausgezogen werden kann, und **dadurch gekennzeichnet, dass** das erste Ende ferner ein Befestigungselement (214; 272) beinhaltet, das so konfiguriert ist, dass es an dem Ballonkatheter proximal beabstandet zu dem Ballon angebracht werden kann.

2. Einkerbungsvorrichtung gemäß Anspruch 1, wobei das zweite Ende eine Führungsdrahtöffnung (193) oder eine Anschlagstruktur (212) beinhaltet, die so konfiguriert ist, dass sie den Ballon daran hindert, vollständig durch sie hindurchzugehen.

3. Ein Kathetersystem, das Folgendes beinhaltet:
einen Ballonkatheter (10), der einen Katheterschaft und einen ausdehnbaren und
kontrahierbaren Ballon (11) beinhaltet, der auf dem Katheterschaft montiert ist; und
eine Einkerbungsvorrichtung (30) gemäß Anspruch 1 oder Anspruch 2, wobei die Einkerbungsvorrichtung über dem Ballon (11) auf dem Katheterschaft zusammengebaut wird, wobei der Ballon durch den Durchbruch (33) hinausgezogen werden kann, nachdem die Einkerbungsvorrichtung auf dem Katheterschaft zusammengebaut worden ist.

4. Kathetersystem gemäß Anspruch 3, wobei der Ballon (11) vor, während und nach dem In-situ-Zuführen des Kathetersystems in das Körperlumen durch den Durchbruch (33) bewegt werden kann.

5. Kathetersystem gemäß Anspruch 3, wobei das zweite Ende eine Anschlagstruktur beinhaltet, durch die mindestens ein Teil des Ballons nicht hindurchgehen kann.

6. Kathetersystem gemäß Anspruch 3, wobei mindestens eines von dem ersten und dem zweiten Ende verschiebbar auf dem Katheterschaft montiert ist.

7. Kathetersystem gemäß Anspruch 3, wobei sich die Einkerbungsstruktur (32) bei der Ausdehnung des Ballons von dem Katheterschaft weg ausdehnt.

8. Kathetersystem gemäß Anspruch 3, wobei die Einkerbungsstruktur (32) mindestens einen Draht beinhaltet, der sich zwischen dem ersten und dem zweiten Ende erstreckt.

9. Kathetersystem gemäß Anspruch 3, wobei die Einkerbungsvorrichtung (30) eine Spitze beinhaltet, die für ein Inneres des Körperlumens nicht verletzend ist.

10. Kathetersystem gemäß Anspruch 9, wobei die Spitze eine Anschlagstruktur beinhaltet, durch die mindestens ein Teil des Ballons nicht hindurchgehen kann.

11. Kathetersystem gemäß Anspruch 3, wobei die Einkerbungsvorrichtung (30) auf dem Katheterschaft mit dem Befestigungselement zusammengebaut ist und wobei das Befestigungselement von dem Katheterschaft nach außen ausdehnbar und nach innen in Richtung des Katheterschafts kontrahierbar ist.

12. Kathetersystem gemäß Anspruch 3, wobei die Einkerbungsvorrichtung (30) auf dem Katheterschaft mit dem Befestigungselement zusammengebaut ist und wobei das Befestigungselement mindestens einen Teil eines Außenumfangs des Katheterschafts umschließt.

13. Kathetersystem gemäß Anspruch 3, wobei die Einkerbungsvorrichtung (30) einen sich proximal erstreckenden Steuerdraht (215) beinhaltet.

14. Kathetersystem gemäß Anspruch 3, wobei die Einkerbungsvorrichtung (30) einen radial ausdehnbaren Abschnitt (217) neben der Einkerbungsstruktur (32) beinhaltet.

## Revendications

1. Un dispositif d'entaillage (30 ; 210) comprenant une structure d'entaillage (32 ; 211) configurée pour entailler une matière à l'intérieur d'une lumière corporelle (101), ledit dispositif d'entaillage comprenant une première extrémité et une deuxième extrémité, où ladite première extrémité comprend une ouverture (33),
où ladite ouverture est configurée pour qu'un ballonnet (11 ; 221) monté sur une tige de cathéter traverse celle-ci jusque dans ladite structure d'entaillage et pour que le ballonnet soit complètement extrait dudit dispositif d'entaillage, et
**caractérisé en ce que** la première extrémité comprend en outre un élément d'assujettissement (214 ; 272) configuré pour s'attacher au cathéter à ballonnet espacé de façon proximale relativement au ballonnet.

2. Le dispositif d'entaillage selon la revendication 1, où ladite deuxième extrémité comprend un orifice pour fil-guide (193) ou une structure formant butée (212) configurés pour empêcher le ballonnet de traverser complètement ceux-ci.

3. Un système de cathéter comprenant :
un cathéter à ballonnet (10) comprenant une tige de cathéter et un ballonnet pouvant se dilater et se contracter (11) monté sur ladite tige de cathéter ; et
un dispositif d'entaillage (30) tel que revendiqué dans la revendication 1 ou la revendication 2, ledit dispositif d'entaillage étant assemblé par-dessus ledit ballonnet (11) sur ladite tige de cathéter, où ledit ballonnet peut être extrait par l'ouverture (33) après que ledit dispositif d'entaillage est assemblé sur ladite tige de cathéter.

4. Le système de cathéter selon la revendication 3, où ledit ballonnet (11) peut être déplacé à travers ladite ouverture (33) avant, pendant et après la mise en place in-situ dudit système de cathéter dans ladite lumière corporelle.

5. Le système de cathéter selon la revendication 3, où ladite deuxième extrémité comprend une structure formant butée à travers laquelle au moins une partie dudit ballonnet ne peut pas passer.

6. Le système de cathéter selon la revendication 3, où au moins une desdites première et deuxième extrémités est montée de façon coulissante sur ladite tige de cathéter.

7. Le système de cathéter selon la revendication 3, où ladite structure d'entaillage (32) se dilate tout en s'éloignant de ladite tige de cathéter au moment de la dilatation dudit ballonnet.

8. Le système de cathéter selon la revendication 3, où ladite structure d'entaillage (32) comprend au moins un fil s'étendant entre lesdites première et deuxième extrémités.

9. Le système de cathéter selon la revendication 3, où ledit dispositif d'entaillage (30) comprend un embout qui n'est pas blessant pour un intérieur de ladite lumière corporelle.

10. Le système de cathéter selon la revendication 9, où ledit embout comprend une structure formant butée à travers laquelle au moins une partie dudit ballonnet ne peut pas passer.

11. Le système de cathéter selon la revendication 3, où ledit dispositif d'entaillage (30) est assemblé sur ladite tige de cathéter à l'aide de l'élément d'assujettissement et où l'élément d'assujettissement peut se dilater vers l'extérieur à partir de ladite tige de cathéter et peut se contracter vers l'intérieur en direction de ladite tige de cathéter.

12. Le système de cathéter selon la revendication 3, où ledit dispositif d'entaillage (30) est assemblé sur ladite tige de cathéter à l'aide de l'élément d'assujettissement et où l'élément d'assujettissement s'enroule autour d'au moins une partie d'une périphérie externe de ladite tige de cathéter.

13. Le système de cathéter selon la revendication 3, où ledit dispositif d'entaillage (30) comprend un fil de commande s'étendant de façon proximale (215).

14. Le système de cathéter selon la revendication 3, où ledit dispositif d'entaillage (30) comprend une section pouvant se dilater radialement (217) adjacente à ladite structure d'entaillage (32).
